# EUROPÄISCHE PATENTSCHRIFT

(11) **EP 2 216 063 B1**
(45) Veröffentlichungstag und Bekanntmachung des Hinweises auf die Patenterteilung: **21.02.2018**
(21) Anmeldenummer: 10400005.4
(22) Anmeldetag: 05.02.2010
(51) Int. Cl.: A61M 16/08, A61M 16/00

(54) **Automatische Komplikationskontrolle**
Automatic complication control
Contrôle de complication automatique

(30) Priorität: 05.02.2009 DE 102009008071
(43) Veröffentlichungstag der Anmeldung: 11.08.2010
(73) Patentinhaber: Löwenstein Medical Technology S.A., 2557 Luxembourg (LU)
(72) Erfinder: Schwaibold, Matthias, 76137 Karlsruhe (DE); Schöller, Bernd, 76135 Karlsruhe (DE)
(74) Vertreter: Klickow & Wetzel PartGmbB

(56) Entgegenhaltungen:
- EP-A1- 1 859 733
- EP-A2- 1 226 841
- WO-A1-98/41267
- WO-A1-2007/041797
- WO-A2-2004/049912
- DE-A1-102007 039 004
- US-A1- 2008 072 896

## Beschreibung

Die Erfindung betrifft eine Vorrichtung zur Beatmung bei sensorischer Erfassung mindestens eines Parameters.

Die Herzinsuffizienz ist die krankhafte Unfähigkeit des Herzens, die vom Körper benötigte Blutmenge (Herzzeitvolumen) ohne Druckanstieg in den Herzvorhöfen zu fördern. Es besteht eine hohe Komorbidität zwischen Herzinsuffizienz und respiratorischen Erkrankungen, insbesondere nächtlichen Atemstörungen. Bei kardiovaskulären Erkrankungen treten die schlafbezogenen Atmungsstörungen (SBAS) in deutlich höherer Prävalenz auf. So findet man bei Herzinsuffizienz mit systolischer Dysfunktion und auch bei der Koronaren Herzkrankheit (KHK) zu 35% eine koprävalent vorliegende obstruktive Schlafapnoe (OSA). Patienten mit Vorhofflimmern und Kardioversion weisen zu 50% OSA auf und Patienten mit Lone Atrial Fibrillation (idiopathisches Vorhofflimmern ohne strukturelle Herzerkrankung) zu 33%. Bei jedem zweiten Betroffenen mit akutem Apoplex liegt gleichzeitig eine obstruktive Schlafapnoe vor. Patienten mit Typ 2 Diabetes sind zu 23% von OSA betroffen.

Die Datenlage zeigt, dass Patienten mit schlafbezogenen Atmungsstörungen und Patienten mit Erkrankungen des Herz-Kreislauf-Systems viele gemeinsame Risikofaktoren aufweisen wie z. B. Übergewicht, gestörter Zucker- und Fettstoffwechsel, Bluthochdruck (jeder Zweite mit Bluthochdruck ist von OSA betroffen). Außerdem sind Überschneidungen in den zugrunde liegenden pathophysiologischen Mechanismen festzustellen. Hierzu gehören die Überaktivität des Sympathikus, die Triggerung systemischer Entzündungsmarker wie CRP und IL-6, eine veränderte Blutgerinnung und die endotheliale Dysfunktion, die allesamt in einen Circulus vitiosus des Herz-Kreislauf-Systems münden.

Die Folgen kardiorespiratorischer Krankheiten zeigen sich in der Kompensation und Dekompensation des Körpers. Die Bestimmung der Kompensation und Dekompensation ist damit ein Maß für den Schweregrad der Erkrankung. Bei der kompensierten Herzinsuffizienz bestehen trotz bereits vorhandener Funktionsstörung des Herzens noch keine Symptome oder sie treten nur bei stärkerer körperlicher Belastung auf. Eine Herzinsuffizienz ist dekompensiert, wenn bereits in Ruhe und ohne Belastung Wasseransammlungen (Ödeme) oder Luftnot (Dyspnoe) auftreten und damit ein hohes Risiko mit verbundener Mortalität besteht.

In der EP 1 226 841 A2 wird bereits eine Vorrichtung zur Beatmung mit Sensoren beschrieben. Die Messwerte der Sensoren ermöglichen eine Ermittlung von Druck, Flow sowie Volumen der betreffenden Gase.

Aus der EP 1 859 733 A1 ist eine Vorrichtung bekannt, die einen aktuellen Zustand eines Patienten anhand von verschiedenen Parametern erfasst und auswertet. Eine Signalisierung erfolgt über eine Warnstufe sowie Alarmstufe.

In der WO 98 41 267 A1 wird bereits eine Vorrichtung zur Beatmung beschrieben, die Sensoren zur Bereitstellung von Messwerten für Druck oder Flow oder Volumen aufweist und bei der sowohl eine Recheneinheit als auch eine Eingabeeinheit verwendet wird, sowie bei der die Recheneinheit sowohl eine Signalauswertung durchführt als auch eine Anzeige unter Verwendung vereinfachter Darstellungen vornimmt.

In der US 2008/007 2896 A1wird eine weitere Vorrichtung zur Beatmung beschrieben, die Sensoren zur Ermittlung von Messwerten von Druck oder Flow oder Volumen aufweist. Auch bei dieser Vorrichtung wird sowohl eine Recheneinheit als auch eine Eingabeeinheit verwendet und die Recheneinheit generiert eine optische Anzeige.

In der vorliegenden Patentanmeldung werden Verfahren und Parameter entwickelt, um den kardiorespiratorischen Krankheitsverlauf zu prädizieren, insbesondere den Schweregrad einer Herzinsuffizienz und die Therapiequalität einer schlafbezogenen Atemstörung. Ziel ist, erste Anzeichen zu erkennen, die auf eine Verschlechterung des Gesundheitszustandes, insbesondere eine kardiale Dekompensation hindeuten, um frühzeitig therapeutische Maßnahmen bei einer Verschlechterung des Zustands einzuleiten.

Die Komorbidität einer Herzinsuffizienz und auch koronarer Herzerkrankungen mit nächtlichen Atmungsstörungen ist insbesondere auch für die Beatmungstherapie interessant, da während der Beatmung cardiorespiratorische Parameter durch das Beatmungsgerät erfassbar sind und dadurch der Patientenzustand ableitbar wird. Damit gelingt eine frühe Erkennung des kardialen Zustands des Patienten. Die Parameter lassen sich somit hervorragend zum Monitoring und Feedback für die suffiziente Beatmungstherapie heranziehen. Wird eine Dekompensation frühzeitig erkannt und verhindert, kann die Mortalität in hohem Maße gesenkt werden. Zudem ist der volkswirtschaftliche Nutzen sehr hoch, da dekompensierte Patienten in der Therapiefolge sehr hohe Kosten nach sich ziehen.

Mehr als 50 % der Patienten mit Herzinsuffizienz der NYHA-Klasse II-IV sind gleichzeitig von einer schlafbezogenen Atmungsstörung betroffen. Circa 30-40 % der Betroffenen zeigen hierbei eine zentrale Atmungsstörung des Cheyne-Stokes-Typs. In der Arbeitsgruppe von Marin et al. [1] konnte gezeigt werden, dass Patienten mit OSA ein erhöhtes Risiko zu kardialen Ereignissen tragen. Dieses Risiko lässt sich durch eine CPAP-Beatmungstherapie (Continous Positive Airway Pressure) signifikant reduzieren.

Anhand der Ergebnisse lässt sich nachweisen, dass unbehandelte OSA-Patienten (AHI > 30) ein etwa um den Faktor 3 erhöhtes Risiko zu fatalen und nicht-fatalen kardialen Ereignissen aufweisen. Schnarcher tragen kein zusätzliches kardiales Risiko.

Die Linksherzinsuffizienz zeichnet sich durch die Dyspnoe (anfangs Belastungs-, später Ruhedyspnoe) oder Orthopnoe aus. Die Orthopnoe äußert sich durch stärkste Atemnot, die einen Einsatz der Atemhilfsmuskulatur in sitzender Körperhaltung nötig macht. Lungenödem, Zyanose, Leistungsminderung und Schwächegefühl sind die Folgen. Bei einer Rechtsherzinsuffizienz sind Venenstauung (Halsvenen, Venen am Zungengrund), Gewichtszunahme und Schwellung des Gewebes aufgrund einer Einlagerung von Flüssigkeit aus dem Gefäßsystem sichtbar. Die Folgen daraus sind schwerer Notfall bis hin zum Tod.

Die Erkennung und Verhinderung einer schleichenden Dekompensation bietet daher die Chance, Notfallereignisse zu vermeiden und die Mortalität zu verringern.

Erfindungsgemäß werden eine Vorrichtung vorgestellt welche in ein Beatmungsgerät zur Therapie von Herzinssufizienz-Patienten oder Patienten mit schlafbezogenen Atemstörungen integrierbar sind.

Für die Dekompensationserkennung werden als Eingangsparameter folgende Größen für die Beurteilung der Leitsymptome verwendet:
▪ Sauerstoffsättigung (SpO2) und Pulswelle entnommen aus einem Pulsoximeter, welches mit einem Beatmungsgerät verbunden sein kann
▪ Atmungskurve und Atemfrequenz (wird über das Beatmungsgerät erfasst)
▪ Diese Parameter werden in ihrem zeitlichen Verlauf analysiert und hinsichtlich des zu ermittelnden Risikos beurteilt. Dabei werden folgende Analysen durchgeführt:
▪ Die Atmung, insbesondere die Atemfrequenz und ihre Variabilität und Atemmuster wie z.B. Cheyne-Stokes-Atmung wird untersucht
▪ Aus der Pulswelle werden Pulsfrequenzvariabilität sowie hämodynamische Größen ermittelt
▪ Die Therapiequalität und die korrekte Anwendung der Therapie schlafbezogener Atemstörungen wird anhand von Parametern wie der Nutzungsdauer der Therapie, der Leckage der Beatmungsmaske und der Anzahl bestimmter Atemereignisse bewertet.

Die Verfahren der Pulsoximetrie, der Atmungs- und Pulswellenanalyse werden miteinander kombiniert, um durch die Verknüpfung der gemessenen Parameter eine beginnende und sich entwickelnde Dekompensation zu erkennen.

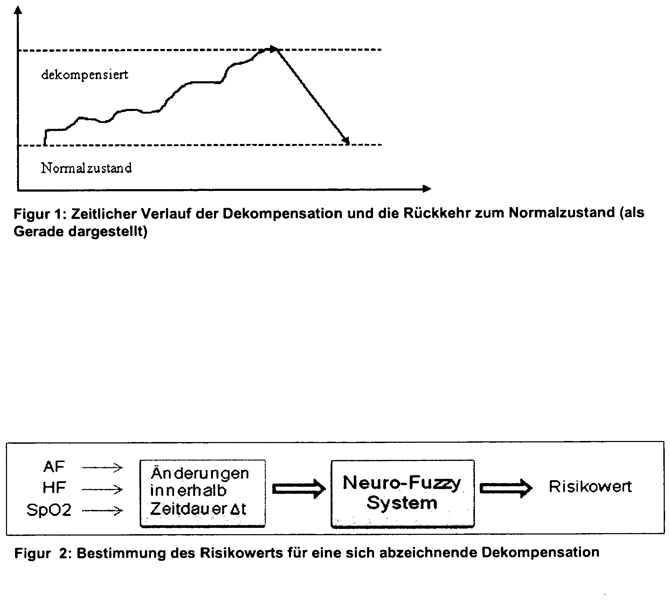

Aus den o.a. Parameterverläufen lässt sich eine Verschlechterung des Patientenstatus bis hin zu einer Dekompensation erkennen. Der Verlauf der Dekompensation ist in Abbildung 1 dargestellt.

Beobachtet man nun den Zustand des Patienten anhand der Parameterverläufe kontinuierlich, so ändern sich die Parameter auf Grund der Zustandsverschlechterung entsprechend des dargestellten Verlaufs. Unbekannt ist die Zeit, in der sich diese Veränderung entwickelt. Über die Art der Veränderungen, ob stetig bzw. sprunghaft, ist nichts bekannt. Auch über das Einsetzen sowie den Verlauf in den Normalzustand sind derzeit keine Untersuchungsergebnisse aus der Literatur bekannt. Die Rückführung in den Normalzustand ist deswegen nur als Gerade eingezeichnet.

Es werden diese Parameter an Patienten mit Herzinsuffizienz erfasst und hinsichtlich des zeitlichen Verlaufs beobachtet mit dem Ziel, prognostischen Aussagen zu erhalten.

Die Erfassung der Vitalsignale erfolgt mittels zweier Sensorsysteme. Die Pulsoximetriesignale werden optisch erfasst. Neben der Messung von
▪ Pulswelle und Pulsfrequenz
▪ Sauerstoffsättigung
▪ Pulswellenamplitudenänderungen als Maß für hämodynamische Änderungen
werden zusätzlich Atmungsverläufe mittels einer nasalen Applikation (Staudruckverfahren für den Einsatz bei den klinischen Untersuchungen) oder aus einem Beatmungsgerät (falls dieses bei Patienten eingesetzt ist) gewonnen:
▪ Daten über die Atmung und die Atemkurven

Die Vitalsignale werden erfasst und durch das Auswerteverfahren unmittelbar analysiert, um die Kenngrößen und ihre zeitliche Veränderung zu berechnen und miteinander über eine Merkmalfusion zu kombinieren sowie hinsichtlich charakteristischer Zustände zu klassifizieren.

Die Erfindung betrifft eine Vorrichtung zur Beatmung, die zum Erfassen und Ausgeben wenigstens eines während der Beatmung zu überwachenden Parameters ausgebildet ist.

Herkömmliche Vorrichtungen zur Beatmung weisen zumeist ein Display auf, um bestimmte aktuelle Informationen für den Benutzer darzustellen. Im einfachsten Fall werden Parameter der Beatmung, wie aktueller Fluss oder Druck der Atemgase, in Form von Zahlenwerten oder in Form einer graphischen Darstellung angezeigt. Daneben sind andere optische Anzeigemöglichkeiten wie Balkendiagramme zur Darstellung von Parametern bekannt.

Die Informationen an den Benutzer sind lediglich Angaben zu den Parametern Druck, Flow, Volumen und Zeit, ohne die Qualität oder Eignung der Parameter für den Behandlungserfolg anzugeben.

Bei bestimmten zu überwachenden Parametern oder bestimmten Beatmungsformen muss der Benutzer des Geräts kontinuierlich einen oder mehrere Parameter überwachen, um den Erfolg der Therapie beurteilen zu können. Dies ist für den behandelnden Arzt meist problemlos durch Interpretation der Daten möglich.

Bei der Beatmung in der Klinik werden vom Beatmungsgerät akustische Alarme ausgegeben, wenn die Beatmung des Patienten insuffizient ist oder ein Gerätedefekt vorliegt. Diese Alarme sind unmittelbare Reaktionen auf eintretende Ereignisse oder Veränderungen die dazu dienen, das Pflegepersonal aufmerksam zu machen.

Ein Patient, der zu Hause beatmet wird, hat hingegen keine Rückmeldung darüber, ob die gewählten Beatmungseinstellungen für ihn geeignet sind. Auch sind im häuslichen Umfeld akustische Alarme nicht hilfreich, weil der Patient nicht adäquat darauf reagieren kann.

Die über das Display dargestellte Information an den Benutzer ist nicht wertend, sondern eine reine Wiedergabe der Ist-Werte. Es wird keine Interpretationshilfe angeboten. Bei der vorliegenden Erfindung handelt es sich um erkannte Zustände / Komplikationen, die nicht kurzfristig kritisch für den Patienten sind, dass also beispielsweise kein akuter Druckabfall oder ein zu niedriges Atemvolumen für einen oder wenige Atemzüge vorliegen. Die Komplikationen sind vielmehr Zustände, die sich aus Parametern erkennen lassen, die über einen längeren Zeitraum bestimmt werden, und daher auch nicht während der Therapie am Display angezeigt werden, sondern im Gerät für ein späteres Auslesen gespeichert werden. Beispiele dafür sind: AHI (Apnoe-Hypopnoe-Index), Häufigkeit von Schnarchen, durchschnittliche Leckage oder ein anderer Wert, der die Häufigkeit von Leckagen beschreibt, Nutzungsdauer, Aufweckreaktionen, Anteil spontaner und mandatorischer Atemzüge, Eingaben des Patienten am MMI (Mensch Maschine Interface) über seine Therapiezufriedenheit.

Der Erfindung liegt daher das Problem zugrunde, eine Vorrichtung zur Beatmung anzugeben, die die Überwachung oder Interpretation eines oder mehrerer Parameter, insbesondere für den medizinischen Laien, erleichtert.

Zur Lösung dieses Problems ist bei einer Vorrichtung zur Beatmung erfindungsgemäß vorgesehen, dass die Vorrichtung zum Bewerten des wenigstens einen erfassten Parameters und zum Ausgeben eines in Abhängigkeit von dem Ergebnis der Bewertung veränderlichen Signals ausgebildet ist.

Erfindungsgemäß repräsentiert das Signal den Grad der Abweichung des Parameters vom Zielwert oder einen Trendwert über das Auftreten oder die Häufigkeit des Auftretens bestimmter Ereignisse bzw. Zustände.

Die Erfindung beruht auf der Erkenntnis, dass ein oder mehrere Parameter, die optisch angezeigt werden, dem Benutzer zusätzlich oder alternativ als Abweichung vom Zielwert vermittelt werden.

Vor der Ausgabe wird der Parameter einer Bewertung unterzogen, anschließend wird das Ergebnis der Bewertung als Signal optisch, mechanisch oder akustisch ausgegeben. Im Rahmen der Bewertung kann es vorgesehen sein, dass die Erfüllung von einer oder mehreren vorgegebenen Bedingungen geprüft wird. Im einfachsten Fall kann geprüft werden, ob der aktuelle Wert des Parameters oberhalb oder unterhalb eines Schwellenwertes liegt. Das Ergebnis dieser Bewertung ist entweder "ja" oder "nein", einem oder beiden Ergebnissen ist ein separates Signal zugeordnet, das anschließend ausgegeben wird.

Dabei ist es wichtig, dass nicht nur die Schwellwerte selbst vorgegeben werden, sondern auch der Zeitraum (die Gesamtzeit), für den sie mindestens über- bzw. unterschritten sein müssen, bevor eine bestimmte Bedingung als erfüllt bewertet wird. Diese Gesamtzeit kann variieren in Abhängigkeit von der Höhe der über- oder Unterschreitung der Schwellwerte.

Die Gesamtzeit ist bevorzugt größer als fünf Atemzüge und besonders bevorzugt größer als zehn Atemzüge und ganz besonders bevorzugt größer als fünfzehn Atemzüge.

Erfindungsgemäß ist die Gesamtzeit, insbesondere bei Anwendungen des Beatmungsgerätes über mehrere Stunden beziehungsweise über Nacht, größer als eine Stunde und besonders bevorzugt größer als drei Stunden und ganz besonders bevorzugt größer als sechs Stunden.

Die ausgewerteten Zeiträume sind somit länger als wenige Atemzüge bzw. wenige Minuten.

Gleichwohl wird die fest einprogrammierte und/oder frei eingegebene Gesamtzeit in Zeitabschnitte unterteilt, wobei in einen Zeitabschnitt zumindest eine Über- oder Unterschreitung eines Schwellwertes fallen kann.

Es liegt auch im Rahmen der Erfindung, dass während der Bewertung ermittelbar ist, ob ein Parameter innerhalb oder außerhalb eines zulässigen Wertebereichs liegt. Dazu wird in Ergänzung der zuvor erwähnten Überprüfung der Überschreitung eines Schwellenwertes geprüft, ob ein zweiter Schwellenwert über- oder unterschritten ist. Die beiden Schwellenwerte bilden obere und untere Grenzen für den Parameter.

Erfindungsgemäß sind unterschiedliche Möglichkeiten vorgesehen, um das Signal bei einem außerhalb des zulässigen Wertebereichs liegenden Parameter zu verändern. Bei einer Variante ist vorgesehen, dass die Dauer des akustischen Signals verändert wird. Diese Veränderung wird sehr leicht bemerkt und als Parameterabweichung interpretiert. Eine andere Variante sieht vor, dass die Lautstärke des akustischen Signals verändert wird. Eine Parameterabweichung kann auf diese Weise durch eine erhöhte Lautstärke signalisiert werden.

Die Erfindung betrifft im Wesentlichen Beatmungsgeräte wie die APAP-Geräte, Bilevel-Geräte, Servoventilations-Geräte, Geräte für die Heimbeatmung und Intensivbeatmung sowie Notfallbeatmungsgeräte, die bereits Atemereignisse und Leckage-Abschnitte erkennen und speichern.
Bei der Einstellung des Beatmungsgerätes gibt der Arzt Schwellenwerte vor und/oder bestimmt aufgrund der durch ihn gewählten Beatmungseinstellungen beispielsweise Obergrenzen und Untergrenzen für die Parameter Druck, Flow, Zeit und Volumen. Ebenso gibt der Arzt die Frequenz der Beatmung vor. Im Rahmen einer Therapie der Schlafapnoe kann der Arzt für den Patienten tolerierbare Schwellenwerte für die Anzahl der Atemaussetzer vorgeben, beispielsweise nicht mehr als 2 Ereignisse pro Stunde für 3 konsekutive Nächte. Bei Überschreitung der vorgegebenen Schwellenwerte generiert das Beatmungsgerät ein Signal und gibt dieses am Display aus. Beispielsweise ist das Signal im Sinne einer Verkehrsampel aufgebaut und leuchtet grün, gelb oder rot. Leuchtet das Signal gelb oder rot, gibt dies dem Patienten ein Feedback, dass er eine Therapiekontrolle und vermutlich eine Veränderung der Therapie benötigt.

Alternativ wird ein bestimmtes Symbol am Gerätedisplay nur dann angezeigt, wenn bestimmte Schwellwerte über- oder unterschritten werden. Es ist außerdem möglich, ein bestehendes Symbol nicht nur in seiner Farbe, sondern auch in seiner Form zu verändern oder von einer dauerhaften Anzeige in eine blinkende Anzeige eines Symbols zu wechseln oder die Blinkfrequenz zu verändern.

Die erfindungsgemäße Anzeige für den Benutzer gibt eine Information über die Qualität der Therapie aus, beispielsweise nach dem Ampelprinzip oder als Grad der Erfüllung der Zieleinstellungen zu 100 %, 90 % ... 50%, 40 % ... 10%, 5%.

Weiterhin ermöglicht es die vorliegende Erfindung, eine Information über den Grad der Zielerreichung für einen vorgebbaren Parameter auszugegeben.

Erfindungsgemäß werden in einer Gesamtbetrachtung zumindest für zwei Parameter aktuelle Werte ermittelt und mit den voreingestellten Zielwerten für diese Parameter verglichen.

Weiterhin kann an den Benutzer eine Information über die Abweichung der aktuellen Werte von den voreingestellten Zielwerten ausgegeben werden.

In einer Weiterbildung der Erfindung sind zumindest zwei Parameter, für die eine Abweichung von den voreingestellten Zielwerten überprüft werden soll, vom Benutzer auswählbar und vorgebbar.

Für den Fall einer Gesamtbetrachtung von zumindest zwei Parametern ist auch vorgesehen, dass aktuelle Werte der ausgewählten Parameter ermittelt und mit den voreingestellten Zielwerten für diese Parameter verglichen werden und eine Zusammenführung der Abweichungen beider Parameter erfolgt und anschließend nur eine Information an den Benutzer ausgegeben wird, die einen Hinweis auf die Abweichung von einem voreingestellten Zielwert gibt, oder die bezogen auf die betrachteten Parameter einen Hinweis auf die Eignung der gewählten Einstellungen der Parameter für die Beatmungstherapie des Patienten gibt.

Erfindungsgemäß erfolgt die Darstellung der Information an den Benutzer beispielsweise auf einem Display. Entsprechend der Auswahl des Benutzers werden auf dem Display nur die ausgewählten zu überwachenden Parameter dargestellt.

Erfindungsgemäß ist vorgesehen, Kontrollergebnisse auf einem Speichermedium zu sichern oder über eine kabelgebundene oder drahtlose Datenübertragungsstrecke an eine auswertende Stelle oder eine Datenbank zu übertragen.

Die erfindungsgemäßen Komplikationen sind Zustände, die sich aus Parametern erkennen lassen, die über einen längeren Zeitraum bestimmt werden. Daher werden die Komplikationsdaten im Gerät für ein späteres Auslesen gespeichert.

Beispiele für Parameter, aus denen Ereignisse, Zustände oder die Therapiequalität allgemein abgeleitet werden, sind: AHI oder AI (Apnoe Index) und/oder zentraler AHI oder AI und/oder obstruktiver AHI oder AI und/oder Auftreten und Dauer von Cheyne-Stokes-Atmung und/oder Häufigkeit von Schnarchen und/oder RERA's (Respiratory Effort-Related Arousals) und/oder reflektorische Verschlüsse der Atemwege und/oder Asynchronie von Beatmung und Atemanstrengung des Patienten und/oder I:E-Verhältnisse und/oder Atemflusskontur und/oder Atemarbeit und/oder Lungenresistance und - compliance und/oder Sekretbildung und/oder Mundatmung und/oder durchschnittliche Leckage und/oder ein anderer Wert, der die Häufigkeit und Stärke von Leckagen beschreibt und/oder Nutzungsdauer und/oder Aufweckreaktionen und/oder Anteil spontaner und mandatorischer Atemzüge und/oder Trends und Verteilungen des Atemvolumens und der Atemfrequenz und/oder Atemstabilität und/oder Trends und Verteilungen der Beatmungsdrücke und/oder Entsättigungen und/oder Veränderungen der Pulsform und/oder rhythmologische Ereignisse des Herzens und/oder Gewicht des Patienten und/oder Trends und Verteilungen der CO2-Konzentration in der Ausatemluft oder im Blut und/oder pH-Wert des Blutes und/oder Schlafstadien und deren Dauer und/oder Eingaben des Patienten am MMI oder per Schnittstelle oder per Speichermedium über seine Therapiezufriedenheit und/oder Veränderungen der Gerätekonfiguration und/oder technische Fehlerzustände des Gerätes und/oder Häufigkeit von Filter- und Maskenwechsel oder allgemeiner Gerätewartung.

Zusätzlich zum Schwellwert, der für eine erkannte Komplikation vorgewählt wird - für einen Parameter oder eine Kombination aus Parametern - kann auch eine Zeitdauer vorgewählt werden, für die der Schwellwert mindestens überschritten sein muss, bevor eine Komplikation erkannt wird. Beispielsweise ein AHI größer x für 3 aufeinanderfolgende Nächte.

Dies gilt auch für die Gesamtbetrachtung von zumindest 2 Parametern.

In einer typischen Ausführungsform werden die Informationen über die Einzelparameter, deren Über- bzw. Unterschreitungen bestimmter Grenzwerte für bestimmte Zeiträume überprüft werden, vor der Rückmeldung an den Anwender zu Aussagen gemäß einem der folgenden Kriterien kombiniert: A) Wirksamkeit der Therapie / Gesundheitszustand allgemein, B) Ausreichende Nutzung des Beatmungsgerätes durch den Patienten, C) Korrekte Nutzung des Beatmungsgerätes durch den Patienten, D) Korrekte Wartung des Gerätes.

Dazu werden über mindestens einen Sensor im Gerät Signale erfasst, vorzugsweise Beatmungsdruck, Atemfluss und/oder Drehzahl der Atemgaspumpe. Aus diesen Signalen erkennt eine Recheneinheit bestimmte Ereignisse, sowie deren Art, Dauer und Häufigkeit.

In die Bewertung über Vergleichsregeln nach den Kriterien-Gruppen A) bis D) fließen typischerweise einzelne der folgenden Einzel-Parameter und Ereignisse ein:

### A)Wirksamkeit der Therapie / Gesundheitszustand allgemein:

- Anzahl zentraler, obstruktiver und gemischter Apnoen innerhalb einer bestimmten Zeiteinheit
- Anzahl zentraler, obstruktiver und gemischter Hypopnoen innerhalb einer bestimmten Zeiteinheit
- Anzahl RERAS oder Schnarchepochen innerhalb einer bestimmten Zeiteinheit
- Anzahl Entsättigungen innerhalb einer bestimmten Zeiteinheit
- mittlere und niedrigste Sauerstoff-Sättigung sowie Anteil unterhalb einer Sättigungs-Grenze innerhalb einer bestimmten Zeiteinheit
- Dauer und Stärke von Cheyne-Stokes-Atmung innerhalb einer bestimmten Zeiteinheit
- Anzahl kortikaler oder autonomer Arousals innerhalb einer bestimmten Zeiteinheit
- Dauer und Stärke einer längerfristigen Hypoventilation, gemessen durch Atem(zeit)volumen-Reduktion und/oder Peakflow-Reduktion und/oder CO2-Anstieg und/oder Sauerstoff-Sättigungs-Abfall

Dabei kann für jeden Parameter einzeln automatisch oder manuell bestimmt werden, welche Werte einer hohen und welche einer niedrigen Wirksamkeit der Therapie entsprechen. Dies kann alternativ auch anstelle der Werte der Parameter selbst über die Stärke der Änderung der Parameter über eine bestimmte Zeitdauer definiert werden. Anschließend wird bevorzugt der stärkste Hinweis auf eine niedrige Therapiequalität als Gesamt-Bewertung der Therapie-Qualität ausgegeben. Dies erfolgt in mindestens 2 Stufen: Qualität niedriq / hoch, typischerweise in 3-10 Stufen.

Unterschreitet die Therapiequalität / der allgemeine Gesundheitszustand einen zuvor festgelegten Schwellwert, wird bevorzugt mindestens eine der folgenden Aktionen durchgeführt:
- optische und/oder akustische Rückmeldung an den Patienten, nach Beendigung des aktuellen Therapieabschnittes. Die optische Rückmeldung erfolgt durch Symbole, z. B. Grafik-Emoticons, und/oder Textausgaben an einem Display des Beatmungsgerätes. Insbesondere ist daran gedacht, in einem Expertenmodus detaillierte Ausgaben über die Ursache der Einschätzung der Wirksamkeit der Therapie darzustellen als im Patientenmodus.
- Bei besonders hoher Gefährdung des Patienten wird zusätzlich ein Alarm während der Therapie erzeugt.
- Speicherung des Zustandes als Ereignis / Marker im internen Datenspeicher oder auf dem Wechselspeichermedium des Gerätes für eine spätere Beurteilung durch geschultes medizinisches Personal.
- Dauerhafte Speicherung (Backup) der Signalverläufe und Ereignisse, die während der kritischen Phase vom Beatmungsgerät aufgezeichnet wurden, im internen Datenspeicher oder auf dem Wechselspeichermedium des Gerätes für eine spätere Beurteilung durch geschultes medizinisches Personal. Insbesondere werden diese Daten vor einem späteren Überschreiben durch neuere Daten geschützt, wie es im Ringspeicher von Beatmungsgeräten sonst üblich wäre.
- Übermittlung des Zustandes über eine Datenverbindung und/oder ein Modem und/oder ein Datennetzwerk an eine Datenbank und/oder geschultes medizinisches Personal.
- Automatische Anpassung der Therapie zur Verbesserung ihrer Wirksamkeit durch das Beatmungsgerät. Dies erfolgt erfindungsgemäß dadurch, dass die Recheneinheit des Beatmungsgerätes in einen speziellen Therapieanpassungsmodus versetzt wird. Im Falle einer CPAP-Therapie oder einer BiLevel-Therapie mit festem IPAP und EPAP ist dieser Anpassungsmodus bevorzugt ein Auto-Titrationsmodus zur Anpassung von CPAP oder IPAP und EPAP für einen begrenzten Zeitraum mit dem Ziel, nach diesem Zeitraum wieder mit festem CPAP oder IPAP und EPAP zu therapieren.
- Empfehlung zu einer ärztlichen Therapiekontrolle oder zu einem Wechsel der Beatmungsform. So erfolgt insbesondere bei CPAP-Therapie eine Empfehlung zu einem Wechsel auf BiLevel-Therapie oder antizyklischer Therapie, wenn zu viele zentrale Apnoen oder Hypopnoen erkannt werden.

### B)Ausreichende Nutzung des Beatmungsgerätes durch den Patienten:

- Anzahl von Tagen und/oder Nächten, an denen die Therapie mindestens für eine definierte Zeitdauer angewendet wurde.
- Anteil von Tagen und/oder Nächten, an denen die Therapie mindestens für eine definierte Zeitdauer angewendet wurde.
- Durchschnittliche Therapiedauer pro Kalender-Tag und/oder Nacht.
- Durchschnittliche Therapiedauer pro Tag und/oder Nacht, an denen die Therapie mindestens für eine definierte Zeitdauer angewendet wurde.

Dabei können bestimmte Bedingungen definiert werden, welche Zeitabschnitte bei eingeschaltetem Beatmungsgerät für die Therapiedauer akzeptiert werden, z. B. Maskenleckage unterhalb eines bestimmten Schwellwertes oder Detektion von Atemzügen oder Verwendung eines Atemluftbefeuchters.

Typischerweise wird ein bevorzugter Parameter für die Bewertung der Nutzung des Beatmungsgerätes durch den Patienten ausgewählt. Über eine Vergleichsregel wird die aktuelle Nutzung mit einem vordefinierten Zielwert für die Nutzung verglichen. Aus diesem Vergleich wird die Nutzung bewertet. Dies erfolgt in mindestens 2 Stufen: Nutzung zu wenig / ausreichend, typischerweise in 3-10 Stufen.

Unterschreitet die Nutzung einen zuvor festgelegten Schwellwert, wird bevorzugt mindestens eine der folgenden Aktionen durchgeführt:
- optische und/oder akustische Rückmeldung an den Patienten.
- Speicherung des Zustandes als Ereignis / Marker im internen Datenspeicher oder auf dem Wechselspeichermedium des Gerätes für eine spätere Beurteilung durch geschultes medizinisches Personal.

- Übermittlung des Zustandes über eine Datenverbindung und/oder ein Modem und/oder ein Datennetzwerk an eine Datenbank und/oder geschultes medizinisches Personal.
- Aktivierung eines veränderten Therapiemodus zur Steigerung des Beatmungskomforts und damit der Nutzung.

### C)Korrekte Nutzung des Beatmungsgerätes durch den Patienten:

- ein Maß für die Dichtigkeit des Patienteninterfaces
- mittlere und/oder durchschnittliche Leckage
- Zeit und/oder Anteil der Zeit mit einer Leckage niedriger als ein vordefinierter Schwellwert
- ein Maß für den korrekten Sitz des Patienteninterfaces
- Verwendung eines erforderlichen Zubehör-Teils, z. B. eines Atemluftbefeuchters oder der Einspeisung von Sauerstoff
- Einhaltung bestimmter erforderlicher Umgebungs-Parameter wie Raumtemperatur, Luftfeuchte etc.
- Einhaltung bestimmter Termine, z. B. Besuche oder Konsultationen bei geschultem medizinischen Personal
- dauerhaft ausreichende Energieversorgung des Beatmungsgerätes.

Typischerweise wird jeder ausgewählte Parameter für die Bewertung der korrekten Nutzung des Beatmungsgerätes ausgewertet. Über eine Vergleichsregel wird jeder Parameter mit einem vordefinierten Zielwert verglichen. Aus diesem Vergleich wird die korrekte Nutzung bewertet. Dies erfolgt in mindestens 2 Stufen: Nutzung hinsichtlich des einzelnen Parameters oder einer Gruppe von Parametern nicht korrekt / korrekt, typischerweise in 3-10 Stufen.

Erfolgt die Nutzung nicht ausreichend korrekt, wird bevorzugt mindestens eine der folgenden Aktionen durchgeführt:
- optische und/oder akustische Rückmeldung an den Patienten.
- Speicherung des Zustandes als Ereignis / Marker im internen Datenspeicher oder auf dem Wechselspeichermedium des Gerätes für eine spätere Beurteilung durch geschultes medizinisches Personal.
- Übermittlung des Zustandes über eine Datenverbindung und/oder ein Modem und/oder ein Datennetzwerk an eine Datenbank und/oder geschultes medizinisches Personal.
- Aktivierung eines veränderten Therapiemodus zur Verbesserung der Therapie speziell für den Zustand der erkannten nicht korrekten Nutzung.

### D)Korrekte Wartung des Gerätes:

- Einhaltung der Intervalle für die Reinigung und/oder den Austausch mindestens eines Luftfilters
- Einhaltung der Intervalle für die Reinigung und/oder den Austausch mindestens eines Patienteninterfaces
- Einhaltung der Intervalle für die Reinigung des Gerätes
- Einhaltung der Intervalle für den Austausch und/oder die Füllung mindestens eines Vorratsbehälters, z. B. einer Flasche mit einem Atemgas, welches eine erhöhte Sauerstoffkonzentration aufweist oder eines Wasserbehälters
- Einhaltung der Intervalle für die Wartung des Beatmungsgerätes
- Einhaltung der Intervalle für die Übermittlung bestimmter Daten, insbesondere aus dem Datenspeicher des Beatmungsgerätes über die korrekte und ausreichende Nutzung sowie über die Wirksamkeit der Therapie und den allgemeinen Gesundheitszustand.
- Beseitigung von erkannten Fehlerzuständen des Beatmungsgerätes oder eines Zubehörteils

Typischerweise wird jeder ausgewählte Parameter für die Bewertung der korrekten Wartung des Beatmungsgerätes ausgewertet. Über eine Vergleichsregel wird jeder Parameter mit einem vordefinierten Zielwert verglichen. Aus diesem Vergleich wird die korrekte Wartung bewertet. Dies erfolgt in mindestens 2 Stufen: Wartung hinsichtlich des einzelnen Parameters oder einer Gruppe von Parametern nicht korrekt / korrekt, typischerweise in 3-10 Stufen.

Erfolgt die Wartung nicht ausreichend korrekt, wird bevorzugt mindestens eine der folgenden Aktionen durchgeführt:
- optische und/oder akustische Rückmeldung an den Patienten.
- Speicherung des Zustandes als Ereignis / Marker im internen Datenspeicher oder auf dem Wechselspeichermedium des Gerätes für eine spätere Beurteilung durch geschultes medizinisches Personal.
- Übermittlung des Zustandes über eine Datenverbindung und/oder ein Modem und/oder ein Datennetzwerk an eine Datenbank und/oder geschultes medizinisches Personal.
- Aktivierung eines veränderten Therapiemodus zur Verbesserung der Therapie speziell für den Zustand der erkannten nicht korrekten Wartung.

Da die Therapie in einem Umfeld stattfindet, welches nicht von medizinischem Fachpersonal überwacht wird, erfolgt nicht notwendigerweise eine akustische oder optische Warnung unmittelbar während der Therapie, um sofort eingreifen zu können. Vielmehr erfolgt eine Ermittlung und Ausgabe der erkannten Komplikationen nach Beendigung eines Therapieabschnittes. Dadurch wird erreicht, dass der Patient sich beim Arzt / Provider meldet - oder der Arzt / Provider bei ihm, im telemedizinischen Fall. Somit ist kein turnusgemäßes Auslesen der Daten aus dem Therapiegerät mehr nötig, eine Reaktion kann bedarfsgesteuert erfolgen.

Erfindungsgemäß ist im Rahmen der Bewertung der Wirksamkeit der Therapie und des Gesundheitszustandes des Patienten auch eine Dekompensationserkennung bei vorliegen einer Herzinsuffizienz vorgesehen.

Dabei erfolgt nicht nur eine Erkennung akuter Komplikationen, sondern auch eine Prädiktion von kritischen Erkrankungszuständen einige Stunden oder Tage bis Wochen vor deren Eintritt.

Dazu werden Information aus Atemfrequenz, Atemvolumen, periodischer Atmung, AHI, Compliance (aus dem Gerät), Sauerstoffsättigung, Herzfrequenz, Pulswellenamplitude, - morphologie, -amplitudenänderungen, Effort, Arousals (aus einem Diagnosegerät) sowie Gewicht, Blutdruck, Beinumfang etc. von extern - über Eingabe am Gerät oder über Schnittstellen zu anderen Geräten - ermittelten oder eingegebenen Daten verwendet.

Die Daten werden durch Kombination der verfügbaren Informationen ("Signalteppich") sowie unter Auswertung des Verlaufes der Daten über einige Minuten, Stunden, einen bis mehrere Tage in einer Signalverarbeitungseinheit, die im Therapiegerät angeordnet sein kann oder auch außerhalb, ausgewertet.

Die Auswertung erfolgt über Vergleichsregeln, bei denen mindestens einer der genannten Parameter und/oder die zeitliche Änderung eines der Parameter mit einem Schwellwert verglichen wird.

In der Gesamtschau erhält der Anwender eine beispielsweise optische oder akustische Rückmeldung über den Grad der Dekompensation.

Ein Leitsymptom für die Dekompensation einer Herzinsuffizienz ist die Kurzatmigkeit und Atemnot. Diese kann durch das Beatmungsgerät bevorzugt über die Atemfrequenz und das Atem(zeit)volumen erkannt werden. Die Atemfrequenz wird in der Recheneinheit des Beatmungsgerätes aus dem Atemfluss beispielsweise über die Anzahl der Inspirationen und Exspirationen innerhalb einer bestimmten Zeiteinheit oder über die Dauer der Inspirationen und Exspirationen ermittelt. Alternativ kann die Atemfrequenz über eine Frequenzanalyse, die Anzahl der Maxima und Minima des Atemflusses, sowie über die Anzahl der Nulldurchgänge des Atemflusses ermittelt werden.

Die Erkennung einer eintretenden oder bevorstehenden Dekompensation kann erfindungsgemäß über die Atemfrequenz derart erfolgen, dass die Atemfrequenz mit einem vordefinierten Schwellwert verglichen wird, um eine Überschreitung des Schwellwertes zu prüfen.

Alternativ ist vorgesehen, dass die Änderung der Atemfrequenz über eine vordefinierte Zeitspanne mit einem vordefinierten Schwellwert verglichen wird, um eine Überschreitung des Schwellwertes zu prüfen.

Außerdem ist bekannt, dass eine Zunahme von Cheyne-Stokes-Atmung ein Anzeichen für eine bevorstehende Dekompensation einer Herzinsuffizienz sein kann. Diese kann durch die Recheneinheit des Beatmungsgerätes bevorzugt über periodisch auftretende Schwankungen im Atemfluss oder Atemvolumen erkannt werden. Alternativ kann die Cheyne-Stokes-Atmung über eine Frequenzanalyse, sowie über Streumaße des Atemflusses oder Atemvolumens oder die Messung der Atemanstrengung ermittelt werden.

Die Stärke der Cheyne-Stokes-Atmung wird erfindungsgemäß über ihre Dauer pro Nacht bestimmt. Alternativ ist daran gedacht, die Stärke der Amplitudenschwankung pro Zyklus (aus Hypo- und Hyperventilation) und die durchschnittliche Zyklusdauer zu berechnen. Die Stärke der Schwankung pro Zyklus wird anschließend mit der Zyklusdauer und/oder Gesamtdauer der Cheyne-Stokes-Atmung multipliziert.

Die Erkennung einer eintretenden oder bevorstehenden Dekompensation kann erfindungsgemäß über die Stärke der Cheyne-Stokes-Atmung derart erfolgen, dass die Stärke der Cheyne-Stokes-Atmung mit einem vordefinierten Schwellwert verglichen wird, um eine Überschreitung des Schwellwertes zu prüfen.

Alternativ ist vorgesehen, dass die Änderung der Stärke der Cheyne-Stokes-Atmung über eine vordefinierte Zeitspanne mit einem vordefinierten Schwellwert verglichen wird, um eine Überschreitung des Schwellwertes zu prüfen.

Ein weiteres Leitsymptom für die Dekompensation einer Herzinsuffizienz ist ein Abfall der Sauerstoffsättigung im Blut. Dieser kann durch ein mit dem Beatmungsgerät verbundenes Pulsoximeter erkannt werden.

Die Erkennung einer eintretenden oder bevorstehenden Dekompensation kann erfindungsgemäß über die Sauerstoffsättigung derart erfolgen, dass die Sauerstoffsättigung mit einem vordefinierten Schwellwert verglichen wird, um eine Unterschreitung des Schwellwertes zu prüfen.

Alternativ ist vorgesehen, dass die Änderung der Sauerstoffsättigung über eine vordefinierte Zeitspanne mit einem vordefinierten Schwellwert verglichen wird, um eine Überschreitung des Schwellwertes zu prüfen.

Außerdem ist bekannt, dass eine Veränderung / Abnahme der Herzfrequenz-Variabilität und/oder autonomen Regulation ein Anzeichen für eine bevorstehende Dekompensation einer Herzinsuffizienz sein kann. Die Herzfrequenz kann durch ein mit dem Beatmungsgerät verbundenes Pulsoximeter oder einem EKG-Sensor ermittelt werden. Die autonome Regulation kann über die Stärke und die Änderung der peripheren Pulsation ermittelt werden, welche ebenfalls über ein Pulsoximeter messbar ist.

Die Erkennung einer eintretenden oder bevorstehenden Dekompensation kann erfindungsgemäß über die Herzfrequenz und deren Variabilität oder die autonome Regulation derart erfolgen, dass die ermittelten Parameter mit je einem vordefinierten Schwellwert verglichen werden, um eine Unterschreitung oder Überschreitung der Schwellwerte zu prüfen.

Alternativ ist vorgesehen, dass die Änderungen der Parameter über eine vordefinierte Zeitspanne mit je einem vordefinierten Schwellwert verglichen werden, um eine Überschreitung des Schwellwertes zu prüfen.

Außerdem ist bekannt, dass eine Wassereinlagerung im Körper ein Anzeichen für eine bevorstehende Dekompensation einer Herzinsuffizienz sein kann. Die Wassereinlagerung kann über den Beinumfang oder über das Gewicht durch eine Waage ermittelt werden.

Die Erkennung einer eintretenden oder bevorstehenden Dekompensation kann erfindungsgemäß über die Wassereinlagerung derart erfolgen, dass die ermittelten Parameter am Beatmungsgerät oder einem separaten Terminal eingegeben und/oder an das Beatmungsgerät über eine Schnittstelle übermittelt werden und mit je einem vordefinierten Schwellwert verglichen werden, um eine Überschreitung der Schwellwerte zu prüfen.

Alternativ ist vorgesehen, dass die Änderungen der Parameter über eine vordefinierte Zeitspanne mit je einem vordefinierten Schwellwert verglichen werden, um eine Überschreitung des Schwellwertes zu prüfen.

Erfindungsgemäß wird bei Auftreten mindestens einer der mit einer Dekompensation in Zusammenhang stehenden Schwellwertüberschreitungen oder -unterschreitungen ein Aktion gemäß der Beschreibung a) ausgeführt. Dabei kann das Beatmungsgerät verschiedene Schweregrade ermitteln und je nach Schweregrad die Aktionen variieren. Insbesondere wird ein Alarm nur bei einem hohen Schweregrad ausgelöst.

Werden mehrere Parameter erfasst, die mit einer Dekompensation in Zusammenhang stehen, werden die einzelnen Parameter und ihr Abstand zu den vordefinierten Schwellwerten über Regeln verknüpft, so dass eine Gesamteinschätzung über das Risiko einer bevorstehenden oder eintretenden Dekompensation vorgenommen wird. Dazu wird bevorzugt Fuzzy-Logik verwendet.

In den Zeichnungen sind Ausführungsbeispiele der Erfindung schematisch dargestellt. Es zeigen:
- Fig. 1: Zeitlicher Verlauf der Dekompensation und die Rückkehr zum Normalzustand
- Fig. 2: Bestimmung des Risikowerts für eine sich abzeichnende Dekompensation
- Fig. 3: einen grundsätzlichen Aufbau einer Vorrichtung zur Beatmung,
- Fig. 4: einen Teilbereich einer Anzeigeeinrichtung,
- Fig. 5: eine Kombination eines Beatmungsgerätes sowie eines Pulsoximeters und
- Fig. 6: ein Beispiel für eine graphische Ausgabe von Auswertungsergebnissen.

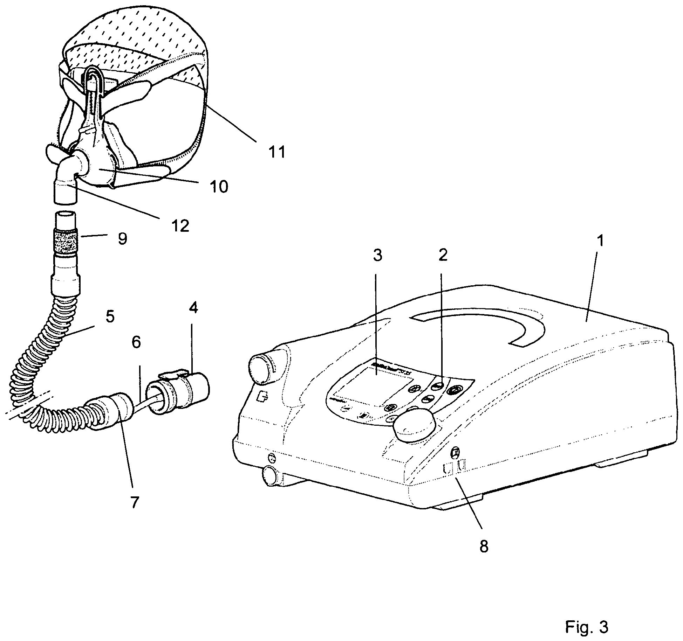

Fig. 3 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Ein Anfeuchter kann adaptiert werden.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 3 zeigt darüber hinaus ein als Beatmungsmaske (10) ausgebildetes Patienten-Interface, das als Nasalmaske realisiert ist. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement(12) auf.

Über die Schnittstelle (8) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Das erfindungsgemäße Beatmungsgerät ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist und eine Einrichtung zur Ermittlung von Druck und/oder Fluss und/oder Volumen des Atemgases, sowie eine Steuereinheit, die so ausgelegt ist, dass sie für jeden Atemzyklus auf der Basis eines vorbestimmten Wertes für den Patienten und/oder auf Basis von Messsignalen für die Parameter Druck und/oder Flow und/oder Volumen einen Atemgasdruck bestimmt und die Quelle für Atemgas derart reguliert, dass der Atemgasdruck erzeugt wird.

Die Steuereinheit ist ferner so ausgelegt, dass sie den aktuellen Druck und/oder Flow und/oder das Volumen von Atemgas bestimmt und den aktuellen Wert über eine mit der Steuereinheit verbundene Anzeige darstellt. Die Steuereinheit ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können.

Weiterhin vergleicht die Steuereinheit solche ParameterWerte, die von einem Benutzer vorgegeben wurden, beispielsweise obere und untere Druckgrenzen oder eine maximal tolerierbare Anzahl von Apnoen pro Zeiteinheit, oder eine maximal tolerierbare Leckage, mit den aktuellen Werten und generiet eine Benutzer-Information zu Abweichungen von der Vorgabe. Die Benutzerinformation wird bevorzugt über das Gerätedisplay graphisch visualiert.

So werden aus dem gemessenen Atemfluss über eine Abnahme des Atem(zeit)volumens für eine Zeitdauer von mindestens 10 s Apnoen und Hypopnoen erkannt. Zusätzlich wird über Druck-und Flowschwankungen Schnarchen erkannt, sowie über die inspiratorische Flowkontur Flattening erkannt. Daraus werden für jede ausreichend lange nächtliche Therapie Indizes berechnet, nämlich: AHI (= Anzahl Apnoen + Hypopnoen pro artefaktfreie Theraiedauer), RDI (= Anzahl aller respiratorischen Ereignisse pro artefaktfreie Therapiedauer), Anteil Atemzüge mit Flattening, Anteil Atemzüge mit Schnarchen.

Für jeden Index kann der Anwender vorwählen, welche Wertebereiche als gut, mittel oder schlechte Therapiequalität eingestuft werden sollen. Zusätzlich kann der Anwender einstellen, wie viele Therapieabschnitte oder Nächte betrachtet werden sollen.

Überschreitet mindestens ein Index die Schwelle für mittlere Therapiequalität, wird die Therapiequalität insgesamt als mittel eingestuft.

Überschreitet mindestens ein Index die Schwelle für schlechte Therapiequalität, wird die Therapiequalität insgesamt als schlecht eingestuft.

Befinden sich alle Indizes im Bereich einer guten Therapiequalität, wird die Therapiequalität insgesamt als gut eingestuft.

Die Bewertung wird dem Patienten durch definierte farbige Symbole am Display angezeigt, erfindungsgemäß insbesondere in den Farben rot, gelb, orange, grün, schwarz, grau und weiß.

Die Symbole sind intuitiv verständlich. Es werden z. B. Ampeln, Grafik-Emoticons oder + und - Zeichen verwendet.

Zusätzlich werden, wenn dies durch den Anwender aktiviert ist, Details über die Ursache der Bewertung der Therapiequalität ausgegeben, z. B. AHI > 10 / h. Außerdem erfolgt eine Empfehlung zu einer ärztlichen Therapiekontrolle oder zu einem Wechsel der Beatmungsform, wenn bestimmte Indizes überschritten werden. So erfolgt insbesondere bei CPAP-Therapie eine Empfehlung zu einem Wechsel auf BiLevel-Therapie oder antizyklischer Therapie, wenn zu viele zentrale Apnoen oder Hypopnoen erkannt werden.

Die Bewertung der Therapiequalität, insbesondere einer mittleren oder schlechten Therapiequalität wird im internen Datenspeicher des Beatmungsgerätes sowie auf einem im Beatmungsgerät befindlichen Wechselspeichermedium gespeichert. Zusätzlich kann die Bewertung über ein Modem in eine Datenbank gespeichert werden.

Zusätzlich zur Darstellung der ermittelten Therapiequalität wird dem Patienten eine Bewertung seiner Nutzungsdauern dargestellt. Dazu wird die mittlere Nutzungsdauer pro Tag / Nacht berechnet. Durch vorgegebene Schwellwerte, z. B. 3 und 5 Stunden, wird diese ebenfalls in die Bereiche gut, mittel und schlecht eingeteilt und über vergleichbare Symbole wie die Therapiequalität am Display dargestellt.

Wird ein Fehler bei der Anwendung oder der Wartung des Beatmungsgerätes erkannt, wird zusätzlich ein entsprechender Hinweis über ein Symbol und/oder einen Text ausgegeben.

Zudem können in einem der Geräte Schnittstellen zu Drittgeräten und Informations-Management-Systemen, beispielsweise zur Aufnahme von Speichermedien, zur Verbindung mit einem EKG, EEG, Drucker, Defibrillator, Pulsoximeter etc., vorgesehen werden.

Über ein Modem oder eine andere Schnittstelle können ebenfalls aufgezeichnete Daten, wie Trends, außerordentliche Ereignisse, Warnmeldungen etc., dem Arzt, sowie Auffälligkeiten, Betriebsstunden oder andere zur Gewährleistung der einwandfreien Funktion dem Wartungs-/Kundendienst bei Bedarf übermittelt werden.

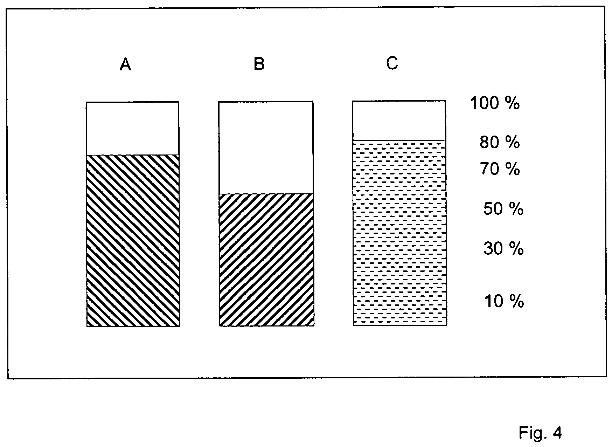

Fig. 4 zeigt einen Teilbereich der Anzeige (3), welcher eine veränderliche Balkenanzeige für die drei Parameter A, B und C wiedergibt. Die Balkenanzeige repräsentiert den Grad der Erfüllung des vorgegebenen Zielwertes. Der Zielwert ist gleich 100% und würde einer voll ausgefüllten Balkenfläche entsprechen. Im Falle des Parameters C ist die aktuell dargestellte Zielerreichung bezogen auf den vorgegebenen Zielwert gleich 80 %.

Die Güte der Erreichung des Zielwertes kann dabei als Abweichung von einem optimalen Wert aufgefasst werden. Im Anschluss an die Bewertung wird beispielsweise über ein Display oder einen Lautsprecher ein Signal ausgegeben. Es kann für den Grad der Abweichung ein zulässiger Wertebereich definiert werden, bei dessen Überschreiten eine Veränderung des Signals eintritt. Der Benutzer erkennt dann eine Signalveränderung als Abweichung von einem Normalwert.

Das Signal kann erfindungsgemäß auf unterschiedliche Arten verändert werden, beispielsweise kann es unterbrochen werden, die Signallänge kann geändert werden, die Signalintensität kann verändert oder es kann eine weitere Signalart hinzugefügt werden. Beispielsweise wird die Balkenanzeige aus Fig. 2 bei Überschreitung des zulässigen Wertebereiches um einen Warnton ergänzt. Dieser Warnton kann erfindungsgemäß bei Überschreitung des zulässigen Wertebereiches zunächst mit einer Frequenz im Bereich 0,2 Hz an- und abschwellen, um dann bei größerer Abweichung vom Zielwert mit steigender Frequenz auf die Abweichung hinzuweisen.

Bezogen auf den voreingestellten Beatmungsdruck erhält der Benutzer eine Information über den aktuellen Druckwert und eine Information über die Abweichung des aktuellen Druckwertes vom voreingestellten Zielwert. Zudem kann die erfindungsgemäße Vorrichtung eine Information an den Benutzer ausgeben, die einen Hinweis auf eine notwendige Anpassung des Beatmungsdruckes gibt.

Bezogen auf den voreingestellten Beatmungsflow erhält der Benutzer eine Information über den aktuellen Flowwert und eine Information über die Abweichung des aktuellen Flowwertes von dem voreingestellten zielwert. Zudem kann die erfindungsgemäße Vorrichtung eine Information an den Benutzer ausgeben, die einen Hinweis auf eine notwendige Anpassung des Beatmungsflows gibt.

Bezogen auf das voreingestellte Beatmungsvolumen erhält der Benutzer eine Information über das aktuell geförderte Volumen an Atemgas und eine Information über die Abweichung des aktuellen Volumens von dem voreingestellten Zielwert. Zudem kann die erfindungsgemäße Vorrichtung eine Information an den Benutzer ausgeben, die einen Hinweis auf eine notwendige Anpassung des Volumens an Atemgas gibt.

Bezogen auf die voreingestellte Beatmungsfrequenz erhält der Benutzer eine Information über die aktuelle Frequenz und eine Information über die Abweichung der aktuellen Frequenz von dem voreingestellten Zielwert. Zudem kann die erfindungsgemäße Vorrichtung eine Information an den Benutzer ausgeben, die einen Hinweis auf eine notwendige Anpassung der Frequenz gibt.

Bezogen auf einen Leckageflow erhält der Benutzer eine Information über das aktuellen Leckageflow und eine Information über die Abweichung des aktuellen Leckageflows von dem voreingestellten Grenzwert. Zudem kann die erfindungsgemäße Vorrichtung eine Information an den Benutzer ausgeben, die einen Hinweis auf eine notwendige Überprüfung der Vorrichtung zur Beatmung, hinsichtlich einer Minimierung bestehender Leckagen gibt.

Bezogen auf die Steilheit des Druckanstieges beim Wechsel von der Expiration in die Inspiration und/oder bezogen auf die Steilheit des Druckabfalls beim Wechsel von der Inspiration in die Expiration erhält der Benutzer eine Information über die aktuelle Steilheit und eine Information über die Abweichung der aktuellen Steilheit von dem voreingestellten Zielwert. Zudem kann die erfindungsgemäße Vorrichtung eine Information an den Benutzer ausgeben, die einen Hinweis auf eine notwendige Anpassung der Steilheit des Druckanstieges und/oder des Druckabfalls gibt.

Bezogen auf voreingestellte Zeiträume, für die beispielsweise der Beatmungsdruck in der Inspiration oder Expiration auf einem definierten Niveau gehalten wird, erhält Benutzer eine Information über den aktuellen Zeitraum für den ein Druck gehalten wird und eine Information über die Abweichung des aktuellen Zeitraumes von dem voreingestellten Zielwert. Zudem kann die erfindungsgemäße Vorrichtung eine Information an den Benutzer ausgeben, die einen Hinweis auf eine notwendige Anpassung des Zeitraumes gibt.

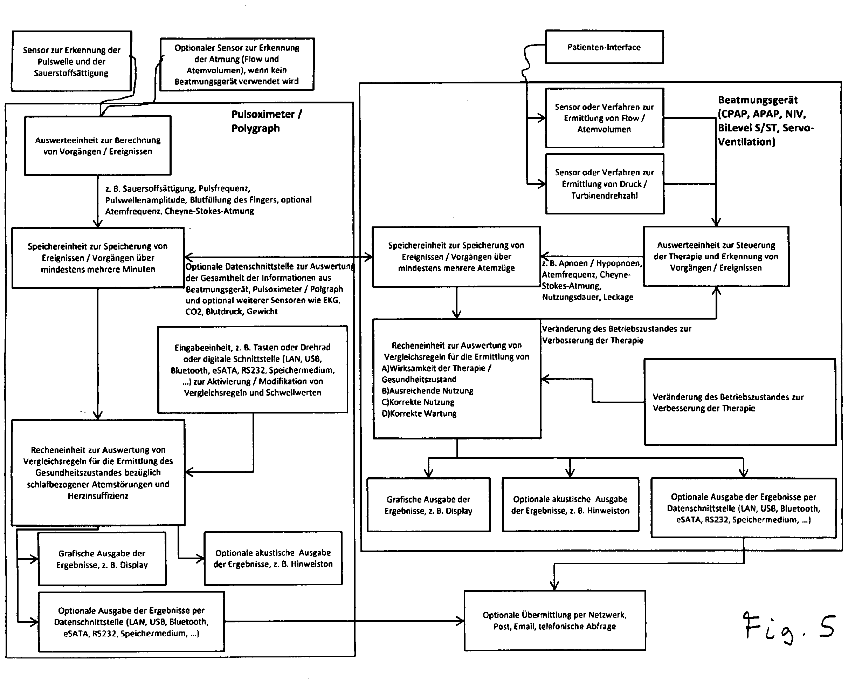

Fig. 5 zeigt das Beatmungsgerät (CPAP, APAP, NIV, BiLevel S/ST, Servo-Ventilation) im Zusammenspiel mit dem Pulsoximeter. Das Beatmungsgerät weist einen Sensor und ein Verfahren zur Ermittlung der Atemgasparameter Flow / Atemvolumen Sensor und ein Verfahren zur Ermittlung der Atemgasparameter Druck / Turbinendrehzahl auf.Eine Auswerteeinheit ermittelt aus den Atemgasparametern Vorgänge / Ereignisse wie z. B. Apnoen / Hypopnoen, Atemfrequenz, Cheyne-Stokes-Atmung, Nutzungsdauer, Leckage. Eine Speichereinheit dient zur Speicherung von Ereignissen / Vorgängen über mindestens zwei Atemzüge.Eine Recheneinheit ermittelt anhand der, von der Auswerteeinheit und/oder Speichereinheit bereitgestellten, Daten über hinterlegte Vergleichsregeln für folgende Entitäten einen Indexwert:
A)Wirksamkeit der Therapie / Gesundheitszustand
B)Ausreichende Nutzung
C)Korrekte Nutzung
D)Korrekte Wartung

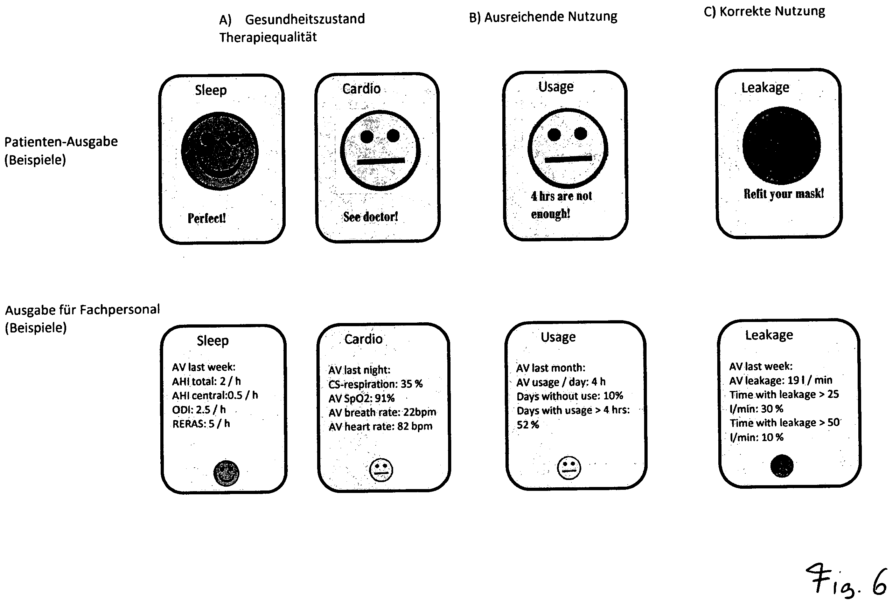

Es erfolgt eine grafische Ausgabe der Ergebnisse wie in Fig. 6 dergestellt, z. B. über ein Display und eineOptionale akustische Ausgabe der Ergebnisse, z. B. Hinweiston. ebenso ist eine Ausgabe der Indexwerte für die Entitäten vorgesehen. Die Informationen über Vorgänge / Ereignisse wird auch unmittelbar zur Steuerung der Atemgaspumpe / des Beatmungsgerätes verwendet. Ziel ist hierbei eine Veränderung des Betriebszustandes zur Verbesserung der Therapie.
optionale Ausgabe der Ergebnisse per Datenschnittstelle (LAN, USB, Bluetooth, eSATA, RS232, Speichermedium, ...) oder eine optionale Übermittlung per Netzwerk, Post, Email, telefonische Abfrage ist alternativ vorgesehen. Das Pulsoximeter oder der PolygraphSensor zur Erkennung der Pulswelle und der Sauerstoffsättigung auf. Ein optionaler weiterer Sensor zur Erkennung der Atmung (Flow und Atemvolumen) ist vorgesehen, wenn kein Beatmungsgerät verwendet wird.Eine Auswerteeinheit ermittelt Vorgängen / Ereignissen wie z.B. Sauersoffsättigung, Pulsfrequenz, Pulswellenamplitude, Blutfüllung des Fingers, optional Atemfrequenz, Cheyne-Stokes-Atmung. Eine Speichereinheit zur Speicherung von Ereignissen / Vorgängen über mindestens zwei Minuten registriert die Vorgänge/Ereignisse.
Über eine optionale Datenschnittstelle zum Beatmungsgerät wird die Gesamtheit der Informationen aus Beatmungsgerät, Pulsoximeter / Polygraph und optional weiterer Sensoren wie EKG, CO2, Blutdruck, Gewicht zusammengeführt. Eine weitere Recheneinheit ermittelt dann aus den Vorgängen / Ereignissen des Beatmungsgerätes und des Pulsoximeters / Polygraphen anhand der, von der Auswerteeinheit und/oder Speichereinheit bereitgestellten, Daten über hinterlegte Vergleichsregeln für folgende Entitäten einen Indexwert:
A)Wirksamkeit der Therapie / Gesundheitszustand
B)Ausreichende Nutzung
C)Korrekte Nutzung
D)Korrekte Wartung

Die Recheneinheit dient ebenso zur Auswertung von Vergleichsregeln für die Ermittlung des Gesundheitszustandes bezüglich schlafbezogener Atemstörungen und Herzinsuffizienz.

Es erfolgt eine grafische Ausgabe der Ergebnisse, z. B. über ein Display und eineOptionale akustische Ausgabe der Ergebnisse, z. B. Hinweiston. ebenso ist eine Ausgabe vorgesehen. Die Informationen über Vorgänge / Ereignisse wird auch unmittelbar zur Steuerung der Atemgaspumpe / des Beatmungsgerätes verwendet.Ziel ist hierbei eine Veränderung des Betriebszustandes zur Verbesserung der Therapie.

Im Rahmen einer Therapie der Schlafapnoe wird die Anzahl der Apnoen pro Stunde überwacht. Bei Überschreitung der durch den Arzt vorgegebenen Schwellenwerte generiert das erfindungsgemäße Beatmungsgerät eine Information an den Benutzer, die einen Hinweis auf eine Überschreitung der Anzahl der Apnoen gibt.

Im einfachsten Fall gibt das Beatmungsgerät daraufhin eine Information an den Benutzer aus, die einen Hinweis auf die Notwendigkeit der Veränderung der Therapieeinstellungen, wie beispielsweise Beatmungsdruck, gibt.

In einer Weiterbildung reagiert das Beatmungsgerät automatisch auf eine erkannte Komplikation und versetzt die Recheneinheit beispielsweise in einen Modus, in dem das Beatmungsgerät einen notwendigen veränderten Beatmungsdruck ermittelt, der geeignet ist, die Anzahl der Apnoen wieder in den voreingestellten Bereich abzusenken und gibt diese Information an den Benutzer aus.

## Patentansprüche

1. Vorrichtung zur Beatmung die zum Erfassen und Ausgeben wenigstens eines, während der Beatmung zu überwachenden Parameters ausgebildet ist, mit Sensoren, deren Messwerte eine Ermittlung von Druck und/oder Flow und/oder Volumen der zugeführten und/oder ausgeatmeten Gase erlauben, einer Recheneinheit, die ausgebildet ist diese Messwerte zu speichern und zur weiteren Berechnungen zu nutzen, einer Eingabeeinheit, durch die in der Recheneinheit hinterlegte Datensätze ausgewählt und/oder in die Recheneinheit externe Daten frei eingegeben werden, die in der Recheneinheit als vorgegebene Schwellwerte, für eine bestimmte Zeit für die sie mindestens über- bzw. unterschritten sein müssen, hinterlegt sind, bevor sie als erfüllt bewertet werden und von der Recheneinheit angewendet werden, wobei die Recheneinheit ferner ausgebildet ist selbsttätig nach einer fest einprogrammierten und/oder frei eingegebenen Vergleichsregel(n) die Messwerte mit den externen Daten in Beziehung zu setzen und dabei eine für die Gesamtzeit gegebene Erfüllung einer Vergleichsregel als Vorgang zu erfassen und abzuspeichern, **dadurch gekennzeichnet, dass** die Recheneinheit ausgebildet ist zunächst zu ermitteln ob die Anzahl der erfassten Vorgänge in der Gesamtzeit jeweils einen bestimmten Wert über- oder unterschreitet, wobei die Recheneinheit ausgebildet ist innerhalb der Gesamtzeit die ermittelten Vorgänge zu differenzieren, hinsichtlich der Höhe der Über-oder Unterschreitung, der Häufigkeit bei denen die Vergleichsregel erfüllt wurde, und mit einem vorgegebenen Muster, gegeben durch die Kombination von entsprechenden Sollwerten, abzugleichen und bei Übereinstimmung in den gegebenen Grenzwerten eine entsprechende, auf das Muster hinweisende, optische Anzeige zu generieren und erst dann die erfassten Vorgänge zu einem Signal zu verarbeiten, und eine entsprechenden optische Anzeige die eine Information über die Qualität der Therapie darstellt zu generieren, und dass eine Ausgabe der Rückmeldung in vereinfachter Form durch Symbole am Gerät für den Patienten erfolgt und dass nach einer Aktivierung durch den Anwender eine Ausgabe der Rückmeldung über die Ursache der Bewertung der Therapiequalität in detaillierter Form für den Experten erfolgt, wobei die Vorrichtung als Beatmungsgerät für Heimanwendungen ausgebildet ist und wobei eine Auswertung der Messergebnisse ausschließlich nach einem Ende der Therapie erfolgt und wobei eine telemetrische Übermittlung der Daten an einen räumlich entfernten Experten durchgeführt wird.

2. Vorrichtung nach Anspruch 1, **dadurch gekennzeichnet, dass** dem Patienten zusätzlich zur Darstellung der ermittelten Therapiequalität eine Bewertung seiner Nutzungsdauer dargestellt wird.

3. Vorrichtung nach wenigstens einem der Ansprüche 1 oder 2, **dadurch gekennzeichnet, dass** die Recheneinheit noch vor Ablauf des Zeitabschnitts, nach dem die Prüfung auf eine Über-/Unterschreitung von Vorgängen gegenüber einem bestimmten Wert erfolgt, auf der Grundlage der bisherigen Vorgänge und ihrer Entwicklung nach einem vorgegebenen Algorithmus eine Prognose für den Rest des Zeitabschnitts berechnet, und wenn nach diesen Werten für den Zeitpunkt des Ablaufs des Zeitabschnitts eine anzuzeigender Vorgang zu erwarten ist, hierauf durch eine entsprechende, insbesondere optische Vorwarnanzeige reagiert.

4. Verfahren zur Darstellung von Messwerten einer Beatmung unter Verwendung der Vorrichtung nach einem der Ansprüche 1 bis 3.

5. Verfahren nach Anspruch 4, **dadurch gekennzeichnet, dass** der Benutzer eine Information darüber erhält, welcher Parameter eine Abweichung vom Zielwert aufweist und welche der Einstellungen Druck, Flow, Volumen, Zeit, Frequenz angepasst werden müssten, um wieder in den Zielbereich zu kommen.

6. Verfahren nach Anspruch 4 oder 5, **dadurch gekennzeichnet, dass** die Informationen über die Zielwertabweichung zusammen mit Empfehlungen hinsichtlich der notwenigen Anpassung eines oder mehrerer Parameter auch im Gerät abgespeichert werden und dort für den Benutzer abrufbar und auf Auswahl hin auch automatisch ausführbar sind.

7. Verfahren nach einem der Ansprüche 4 bis 6, **dadurch gekennzeichnet, dass** die Informationen über die Zielwertabweichung zusammen mit Empfehlungen hinsichtlich der notwenigen Anpassung eines oder mehrerer Parameter auch vom Gerät über Fernübertragung zu einem Arzt übermittelt werden, woraufhin dieser optional ebenfalls über eine Fernübertragung die notwendigen Einstellungen der Parameter anpasst.

8. Verfahren nach einem der Ansprüche 4 bis 7, **dadurch gekennzeichnet, dass** der Schwellwert für zumindest einen Parameter und eine Zeitdauer von zumindest 30 Minuten vorgegeben werden.

9. Verfahren nach einem der Ansprüche 4 bis 8, **dadurch gekennzeichnet, dass** die Informationen über die Einzelparameter, deren Über- bzw. Unterschreitungen bestimmter Grenzwerte für bestimmte Zeiträume überprüft werden, vor der Rückmeldung an den Anwender zu Aussagen gemäß einem der folgenden Kriterien kombiniert werden: A) Wirksamkeit der Therapie / Gesundheitszustand allgemein, B) Ausreichende Nutzung des Beatmungsgerätes durch den Patienten, C) Korrekte Nutzung des Beatmungsgerätes durch den Patienten, D) Korrekte Wartung des Gerätes.

10. Verfahren nach Anspruch 9, **dadurch gekennzeichnet, dass** über mindestens einen Sensor im Gerät Signale erfasst werden, vorzugsweise Beatmungsdruck, Atemfluss und/oder Drehzahl der Atemgaspumpe.

11. Verfahren nach Anspruch 10, **dadurch gekennzeichnet, dass** aus diesen Signalen eine Recheneinheit bestimmte Ereignisse, sowie deren Art, Dauer und Häufigkeit erkennt.

12. Verfahren nach einem der Ansprüche 4 bis 11, **dadurch gekennzeichnet, dass** eine Gesamt-Bewertung mindestens eines der Kriterien in mindestens 2 Stufen erfolgt, vorzugsweise in 3 - 10 Stufen.

13. Verfahren nach einem der Ansprüche 4 bis 12, **dadurch gekennzeichnet, dass** über eine Vergleichsregel ein aktueller Wert mindestens eines Kriteriums mit einem vordefinierten Zielwert verglichen wird.

## Claims

1. Apparatus for ventilation, which is embodied to capture and output at least one parameter to be monitored during the ventilation, said apparatus having sensors, the measurement values of which facilitate an ascertainment of pressure and/or flow and/or volume of the supplied and/or exhaled gases, a computer unit which is embodied to store these measurement values and use said measurement values for further calculations, an input unit, by means of which the data records stored in the computer unit are selected and/or external data is freely entered into the computer unit, said data being stored in the computer unit as predetermined thresholds for a specific length of time during which they must be at least overshot or undershot before they are evaluated as satisfied and applied by the computer unit, wherein the computer unit is further embodied to independently relate the measurement values to the external data according to a fixedly programmed and/or freely entered comparison rule or rules and, in the process, capture and store as a process a comparison rule being satisfied for the entire time, **characterized in that** the computer unit is embodied to initially ascertain whether the number of captured processes during the overall time in each case overshoots or undershoots a certain value, wherein the computer unit is embodied to differentiate, within the overall time, the ascertained processes in respect of the level of overshoot or undershoot and in respect of the frequency with which the comparison rule was satisfied, and compare this to a predetermined pattern, given by the combination of appropriate intended values, and generate an appropriate optical display indicating the pattern in the case of correspondence in the provided limits and only then process the captured processes into a signal and generate a corresponding optical display which represents information about the quality of the therapy and **in that** the feedback is output for the patient in a simplified form by symbols on the device and **in that**, after an activation by the user, an output of the feedback about the cause of the evaluation of the therapy quality is effectuated in detailed form for the expert, wherein the apparatus is embodied as a ventilation device for home applications and wherein the measurement results are only evaluated after an end of the therapy and wherein telemetric transfer of the data to a spatially separated expert is carried out.

2. Apparatus according to Claim 1, **characterized in that**, in addition to the ascertained therapy quality, an evaluation of his use duration is presented to the patient.

3. Apparatus according to at least one of Claims 1 and 2, **characterized in that**, even before the time interval, after which there is the examination in respect of an overshoot/undershoot of processes in relation to a certain value, expires, the computer unit calculates a prediction for the remainder of the time interval on the basis of the previous processes and their development according to a predetermined algorithm and, if a process that is to be displayed is to be expected according to these values for the time when the time interval expires, said computer unit reacts to this by way of an appropriate early warning display, in particular an optical early warning display.

4. Method for presenting measurement values of ventilation using the apparatus according to any one of Claims 1 to 3.

5. Method according to Claim 4, **characterized in that** the user obtains information about which parameter has a deviation from the target value and which of the pressure, flow, volume, time and frequency settings has to be adapted in order to return to the target range.

6. Method according to Claim 4 or 5, **characterized in that** the information about the target value deviation is also stored in the device together with recommendations in respect of the necessary adaptation of one or more parameters and said information can be recalled from there by the user and can also be carried out automatically upon selection.

7. Method according to any one of Claims 4 to 6, **characterized in that** the information about the target value deviation is also transmitted by the device to a medical practitioner by way of a remote control together with recommendations in respect of the necessary adaptation of one or more parameters, whereupon said medical practitioner optionally adapts the necessary settings of the parameters, likewise by way of a remote control.

8. Method according to any one of Claims 4 to 7, **characterized in that** the threshold for at least one parameter and a time duration of at least 30 minutes are predetermined.

9. Method according to any one of Claims 4 to 8, **characterized in that** information about the individual parameters, the overshoot or undershoot of specific limits thereby being monitored for specific periods of time, is combined to statements according to one of the following criteria before the feedback to the user: A) effectiveness of the therapy/health state in general, B) sufficient use of the ventilation device by the patient, C) correct use of the ventilation device by the patient, D) correct servicing of the device.

10. Method according to Claim 9, **characterized in that** signals, preferably ventilation pressure, respiratory flow and/or rotational speed of the respiratory gas pump, are captured by way of at least one sensor in the device.

11. Method according to Claim 10, **characterized in that** a computer unit identifies certain events, and the type, duration and frequency thereof, from these signals.

12. Method according to any one of Claims 4 to 11, **characterized in that** an overall evaluation of at least one of the criteria is effectuated in at least 2 stages, preferably in 3-10 stages.

13. Method according to any one of Claims 4 to 12, **characterized in that** a current value of at least one criterion is compared to a predefined target value by way of a comparison rule.

## Revendications

1. Dispositif d'assistance respiratoire, conçu pour enregistrer et émettre au moins un paramètre à contrôler pendant la respiration, doté de capteurs, dont les valeurs mesurées permettent la détermination de la pression et/ou du débit et/ou du volume de gaz insufflé et/ou expiré, une unité de calcul conçue pour enregistrer ces valeurs mesurées et les utiliser pour des calculs supplémentaires, une unité de saisie, au moyen de laquelle les enregistrements déposés dans l'unité de calcul sont sélectionnés et/ou des données externes sont entrées librement dans l'unité de calcul, qui sont déposées dans l'unité de calcul en tant que valeurs de seuil définies, devant être dépassées ou sous-dépassées pour au moins une durée minimale déterminée, avant d'être évaluées comme satisfaites et appliquées par l'unité de calcul, dans lequel l'unité de calcul est également conçue pour mettre automatiquement en relation les valeurs mesurées avec les données externes selon une règle de comparaison préprogrammée ou saisie manuellement et ainsi enregistrer et mémoriser comme opération le respect d'une règle de comparaison donnée pour la durée totale, **caractérisé en ce que** l'unité de calcul est conçue pour d'abord déterminer si le nombre d'opérations enregistrées sur la durée totale dépasse ou sous-dépasse une valeur définie, dans lequel l'unité de calcul est conçue pour différencier les opérations déterminées dans les limites de la durée totale en ce qui concerne la valeur du dépassement ou du sous-dépassement et la fréquence à laquelle la règle de comparaison est satisfaite, comparer avec un modèle prédéfini, obtenu par combinaison des valeurs obligatoires correspondantes, générer un affichage optique indicatif pour le modèle en cas de conformité avec les valeurs limites données, traiter les opérations enregistrées en un signal et générer un affichage optique correspondant fournissant une information sur la qualité de la thérapie, **en ce qu'**une émission de la réponse sous forme simplifiée au moyen de symboles sur l'appareil en résulte pour le patient et **en ce qu'**après une activation par l'utilisateur une émission de la réponse sur la raison de l'évaluation de la qualité de la thérapie en résulte pour l'expert sous forme détaillée, dans lequel le dispositif est conçu comme appareil respiratoire pour utilisation domestique et dans lequel une évaluation des résultats de mesure résulte exclusivement après une fin de thérapie et dans lequel une transmission télémétrique des données vers un expert éloigné géographiquement est exécutée.

2. Dispositif selon la revendication 1, **caractérisé en ce qu'**une évaluation de la durée d'utilisation est également affichée pour le patient afin d'afficher la qualité déterminée de la thérapie.

3. Dispositif selon l'une des revendications 1 ou 2, **caractérisé en ce que** l'unité de calcul, avant échéance de la période de temps après laquelle il est vérifié si un dépassement ou sous-dépassement par rapport à une valeur définie s'est produit lors des opérations, calcule selon un algorithme un pronostic pour le reste de la période de temps sur la base des opérations précédentes et de leur développement, et réagit ensuite par un affichage d'avertissement correspondant, en particulier optique, lorsqu'une opération à afficher est attendue d'après ces valeurs pour le moment de l'échéance de la période de temps.

4. Procédé pour la réalisation de valeur de mesures d'une respiration en utilisant le dispositif selon l'une des revendications 1 à 3.

5. Procédé selon la revendication 4, **caractérisé en ce que** l'utilisateur obtient une information sur : quel paramètre présente un écart par rapport à la valeur cible et lequel parmi les paramètres de pression, débit, volume, temps et fréquence doit être ajusté pour revenir dans l'intervalle cible.

6. Procédé selon l'une des revendications 4 et 5, **caractérisé en ce que** les informations sur l'écart par rapport à la valeur cible sont enregistrées dans l'appareil en même temps que des recommandations concernant l'ajustement nécessaire d'un ou plusieurs paramètres, sont récupérables par l'utilisateur et peuvent être effectuées manuellement ou automatiquement.

7. Procédé selon l'une des revendications 4 à 6, **caractérisé en ce que** les informations sur l'écart par rapport à la valeur cible peuvent être communiquées par télétransmission à un médecin depuis l'appareil en même temps que des recommandations concernant l'ajustement nécessaire d'un ou plusieurs paramètres, suite à quoi le réglage nécessaire du paramètre est effectué par télétransmission éventuellement et de façon optionnelle.

8. Procédé selon l'une des revendications 4 à 7, **caractérisé en ce que** la valeur de seuil pour au moins un paramètre et une durée d'au moins 30 minutes sont définis.

9. Procédé selon l'une des revendications 4 à 8, **caractérisé en ce que** les informations sur les paramètres unitaires, dont les dépassements ou sous-dépassements de valeurs limites définies pour des durées définies sont vérifiés, sont combinées avant réponse à l'utilisateur sous forme de déclaration selon l'un des critères suivants : A) efficacité de la thérapie / état de santé général, B) utilisation suffisante de l'appareil d'assistance respiratoire par le patient, C) utilisation correcte de l'appareil d'assistance respiratoire par le patient, D) entretien correct de l'appareil.

10. Procédé selon la revendication 9, **caractérisé en ce que** les signaux sont enregistrés par l'appareil au moyen d'au moins un capteur, de préférence la pression de ventilation, le débit expiratoire et/ou la vitesse de rotation de la pompe à gaz respiratoire.

11. Procédé selon la revendication 10, **caractérisé en ce qu'**une unité de calcul reconnaît des événements déterminés à partir de ces signaux, ainsi que leur type, leur durée et leur fréquence.

12. Procédé selon l'une des revendications 4 à 11, **caractérisé en ce qu'**une évaluation totale d'au moins un des critères se fait en au moins deux étapes, de préférence en 3 à 10 étapes.

13. Procédé selon l'une des revendications 4 à 12, **caractérisé en ce qu'**une valeur actuelle d'au moins un critère est comparée avec une valeur cible prédéfinie selon une règle de comparaison.
